# EUROPEAN PATENT SPECIFICATION

(11) **EP 2 497 361 B1**
(45) Date of publication and mention of the grant of the patent: **20.08.2014**
(21) Application number: 10828307.8
(22) Date of filing: 04.11.2010
(51) Int. Cl.: A01K 67/00, C12P 13/00, G01N 33/50, G01N 33/94

(54) **METHOD FOR GRAVITATIONAL FLUCTUATION STRESS LOADING**
VERFAHREN FÜR SCHWERKRAFTFLUKTUATIONSSTRESSBELASTUNG
PROCÉDÉ POUR IMPOSER UN STRESS PAR FLUCTUATION GRAVITATIONNELLE

(30) Priority: 04.11.2009 JP 2009253133
(43) Date of publication of application: 12.09.2012
(73) Proprietor: Mitsubishi Heavy Industries, Ltd., Tokyo 108-8215 (JP)
(72) Inventor: OCHIAI, Toshimasa, Tokyo 108-8215 (JP); YOSHIOKA, Mitsuhiro, Sapporo-shi Hokkaido 060-8638 (JP); GYOTOKU, Junichiro, Kobe-shi Hyogo 652-0863 (JP); OHTA, Hisashi, Ibaraki 305-0024 (JP)
(74) Representative: Bongiovanni, Simone
(86) International application number: PCT/JP2010/069598
(87) International publication number: WO 2011/055747

(56) References cited:
- STEFAN SCHNEIDER ET AL: "Parabolic flight experience is related to increased release of stress hormones", EUROPEAN JOURNAL OF APPLIED PHYSIOLOGY, SPRINGER, BERLIN, DE, vol. 100, no. 3, 10 March 2007 (2007-03-10), pages 301-308, XP019517322, ISSN: 1439-6327, DOI: 10.1007/S00421-007-0433-8
- MONICA S DA SILVA ET AL: "Anorexia in space and possible etiologies", NUTRITION, vol. 18, no. 10, 1 October 2002 (2002-10-01), pages 805-813, XP055053676, ISSN: 0899-9007, DOI: 10.1016/S0899-9007(02)00915-2
- S BLANC ET AL: "Counteraction of spaceflight-induced changes in the rat central serotonergic system by adrenalectomy and corticosteroid replacement", NEUROCHEMISTRY INTERNATIONAL, vol. 33, no. 4, 1 October 1998 (1998-10-01), pages 375-382, XP055053677, ISSN: 0197-0186, DOI: 10.1016/S0197-0186(98)00042-4
- DATABASE MEDLINE [Online] US NATIONAL LIBRARY OF MEDICINE (NLM), BETHESDA, MD, US; May 2001 (2001-05), CHEN X P ET AL: "[Effects of simulated microgravity on the contents of monoamine transmitters in human plasma during heat stress].", XP002692318, Database accession no. NLM21171415 & ZHONGGUO YING YONG SHENG LI XUE ZA ZHI = ZHONGGUO YINGYONG SHENGLIXUE ZAZHI = CHINESE JOURNAL OF APPLIED PHYSIOLOGY MAY 2001, vol. 17, no. 2, May 2001 (2001-05), pages 182-185, ISSN: 1000-6834
- MILLER J D ET AL: "Effects of microgravity on brain neurotransmitter receptors", EUROPEAN JOURNAL OF PHARMACOLOGY, ELSEVIER SCIENCE, NL, vol. 161, no. 2-3, 28 February 1989 (1989-02-28), pages 165-171, XP025545411, ISSN: 0014-2999, DOI: 10.1016/0014-2999(89)90839-X [retrieved on 1989-02-28]
- YOSHIOKA MITSUHIRO: "Overview: Pharmacological potential of microgravity condition", JOURNAL OF PHARMACOLOGICAL SCIENCES, vol. 106, no. Suppl. 1, 2008, page 42P, XP008160239, & 81ST ANNUAL MEETING OF THE JAPANESE-PHARMACOLOGICAL-SOCIETY; YOKOHAMA, JAPAN; MARCH 17 -19, 2008 ISSN: 1347-8613
- MITSUHIRO YOSHIOKA ET AL: "Effects of Parabolic Flight on Serotonin-Related Gene Expression in the Mouse Brain", GRAVITATIONAL AND SPACE BIOLOGY, vol. 26, no. 2, 1 October 2012 (2012-10-01), pages 22-29, XP055053679,
- KIYOAKI KATAHIRA ET AL.: 'Posture analysis in the rat exposed to microgravity during parabolic flight' BIOLOGICAL SCIENCES IN SPACE vol. 13, no. 3, September 1999, pages 172 - 173, XP008156443
- TAKASHI OHIRA ET AL.: 'Neural and/or Mechanical Response of Adductor Longus Muscle to Exposure to Microgravity in Wistar Hannover Rats' JAPANESE JOURNAL OF AEROSPACE AND ENVIRONMENTAL MEDICINE vol. 46, no. 2, 01 June 2009, pages 21 - 28, XP008156748
- YOSHINOBU OHIRA ET AL.: 'Role of Gravity in Mammalian Development: Effect of Hypergravity and/or Microgravity on the Development of Skeletal Muscles' BIOLOGICAL SCIENCES IN SPACE vol. 18, no. 3, November 2004, pages 124 - 125, XP008156444

## Description

### Technical Field

The present invention relates to a method for promoting serotonin-producing gene expression and an efficacy-measuring method.

### Background Art

A known stress loading method in the related art includes a method for placing an acute stress load on a laboratory animal by inducing acrophobia using an elevated plus-maze or an elevated platform (refer to, for example, Non Patent Literature 1 and Non Patent Literature 2).

A biological reaction triggered in the laboratory animal with such an elevated plus-maze is known to be an emotional reaction caused by stress, such as discomfort and fear (refer to, for example, Non Patent Literature 1).

This elevated-platform testing method is also a known method for screening antianxiety agents. Furthermore, the relationship between resistance to stress and disease, such as diabetes, has also been studied using the above-described elevated-platform testing method (refer to, for example, Non Patent Literature 2). Further known stress loading methods are disclosed in Non Patent Literature 3, 4, 5, 6, 7, 8 and 9.

### Citation List

### Non Patent Literature

### {NPL 1}

Taku Yamaguchi et al. "Evaluation of anxiety-related behavior in elevated plus-maze test and its applications," Folia Pharmacologica Japonica, 2005, 126(2) p.99-105

### {NPL 2}

Shigeo Miyata et al. "Elevated-platform testing method: simple behavioral analysis method suitable for stress vulnerability," Folia Pharmacologica Japonica, 2008, 132(4) p. 213-216

### {NPL 3}

Stefan Schneider et al. "Parabolic flight experience is related to increased release of stress hormones", European Journal Of Applied Physiology, 2007, vol. 100, no. 3, p. 301-308

### {NPL 4}

Monica S Da Silva et al. "Anorexia in space and possible etiologies", Nutrition, 2002, vol. 18, no. 10, p. 805-813 {NPL 5}

S Blanc et al. "Counteraction of spaceflight-induced changes in the rat central serotonergic system by adrenalectomy and corticosteroid replacement", Neurochemistry International, 1998, vol. 33, no. 4, p. 375-382

### {NPL 6}

DATABASE MEDLINE [Online] US NATIONAL LIBRARY OF MEDICINE (NLM), BETHESDA, MD, US; May 2001 (2001-05), Chen X P et al. "Effects of simulated microgravity on the contents of monoamine transmitters in human plasma during heat stress", Database accession no. NLM21171415 and Zhongguo Ying Yong Sheng Li Xue Za Zhi = Zhongguo Yingyong Shenglixue Zazhi = CHINESE JOURNAL OF APPLIED PHYSIOLOGY MAY 2001, vol. 17, no. 2, May 2001 (2001-05), pages 182-185 {NPL 7}

Miller J D et al. "Effects of microgravity on brain neurotransmitter receptors", European Journal Of Pharmacology, 1989, vol. 161, no. 2-3, p. 165-171

### {NPL 8}

Yoshioka Mitsuhiro "Overview: Pharmacological potential of microgravity condition", Journal Of Pharmacological Sciences, vol. 106, no. Suppl. 1, 2008, p. 42P, and 81st Annual Meeting Of The Japanese-Pharmacological-Society; Yokohama, Japan; March 17 -19, 2008

### {NPL 9}

Mitsuhiro Yoshioka et al. "Effects of Parabolic Flight on Serotonin-Related Gene Expression in the Mouse Brain", Gravitational And Space Biology, 2012, vol. 26, no. 2, p. 22-29

### Summary of Invention

### Technical Problem

However, with the stress loading method using an elevated plus-maze described in Non Patent Literature 1, it was not possible to selectively secrete serotonin, in other words, to specifically express a serotonin-producing gene, because this stress loading method promoted intracerebral release of not only serotonin but also a number of amino acids, such as dopamine and GABA (Gamma-Amino Butyric Acid).

Furthermore, although the stress loading method using an elevated platform described in Non Patent Literature 2 demonstrated that a biological reaction resulting from acute stress loaded as described above had some relationship with the serotonin nervous system, it did not succeed in offering a specific approach for elucidating the mechanism of such a relationship.

The present invention has been conceived in light of these circumstances and, an object thereof is to provide a method for promoting serotonin-producing gene expression and an efficacy-measuring method.

### Solution to Problem

In order to solve the above-described problems, a method for promoting serotonin-producing gene expression and an efficacy-measuring method according to the present invention provide the following solutions.

In particularthe present invention provides for:
1. A method of promoting gene expression of Tph2, Maob, Sert and S1c7a5 in a mid-brain of a non-human laboratory animal comprising performing a gravitational stress loading method
   which comprises:
   at least one first stress-loading step of placing a stress load on the non-human laboratory animal by means of microgravity, and
   at least one second stress-loading step of placing a stress load on the non-human laboratory animal by means of hypergravity.
2. The method according to point 1, wherein the first stress-loading step and the second stress-loading step are repeated alternately.
3. The method according to points 1 or 2, wherein the first stress-loading step is performed not more than 20 times in one hour.
4. The method according to point 1 wherein a microgravity environment and hypergravity environment are formed in an aircraft through a parabolic flight.
5. An efficacy-measuring method using the method according to one of points 1 to 4, comprising:
   a stress-response producing step of producing a stress response in the non-human laboratory animal by performing the first stress-loading step and the second stress-loading step; and
   a drug screening step.

More specifically, a first aspect according to the present disclosure is a gravitational fluctuation stress loading method including at least one first stress-loading step of placing a stress load on a subject or a laboratory animal by means of microgravity.

According to the gravitational fluctuation stress loading method of the first aspect of the present disclosure, it is possible to apply physical irritation by means of microgravity (µG), which is impossible to produce on the ground, to the subject or the laboratory animal. Microgravity is gravity less than about 1 G. For example, parabolic flight of an aircraft produces about 10⁻² G gravity. The above-described microgravity is preferably maintained at about 3×10⁻² G or less.

The inventors have found that the above-described stress loading method by means of microgravity causes a new stress response, which was impossible to produce with conventional stress loading methods. The above-described new stress response is an acute stress response caused as a result of the central nervous system being stimulated by a specific pathway, in other words, specific to serotonin.

The inventors have also found that the gravitational fluctuation stress loading method according to the present disclosure selectively expresses the genetic system that works so as to produce/retain serotonin, serving as a neurotransmitter.

Furthermore, according to the gravitational fluctuation stress loading method of the first aspect of the present disclosure, it is possible to produce a stress-response producing model that causes the above-described new stress response. With this stress-response producing model, it is possible to search for new drug target molecules that act on the central nervous system related to serotonin.

Furthermore, according to the gravitational fluctuation stress loading method of the first aspect of the present disclosure, the above-described stress-response producing model can be used as a tool for elucidating the mechanism causing panic disorder and anxiety disorder of the central nervous system, which are diseases allegedly related to serotonin.

The gravitational fluctuation stress loading method according to the first aspect of the present disclosure includes at least one second stress-loading step of placing a stress load on the subject or the laboratory animal by means of hypergravity.

In this manner, physical irritation in the form of gravitational fluctuation of microgravity and hypergravity, which are impossible to produce on the ground, can be applied to the subject or the laboratory animal. Hypergravity is gravity more than 1 G, for example, from 1 G to 2.5 G and refers to a state of gravity larger than normal gravity. The above-described hypergravity is preferably maintained at about 1.4 G or less.

In the gravitational fluctuation stress loading method according to the first aspect of the present disclosure, the first stress-loading step and the second stress-loading step may be repeated alternately.

In this manner, alternate physical irritation in the form of gravitational fluctuation of microgravity and hypergravity, which are impossible to produce on the ground, can be applied to the subject or the laboratory animal.

In the gravitational fluctuation stress loading method according to the first aspect of the present disclosure, the first stress-loading step may be performed not more than 20 times in one hour. The first stress-loading step is performed preferably at least eight times.

In this manner, because stress loading by means of microgravity can be ensured at least for about 15 to 20 seconds during one first stress-loading step, sufficient stress loading under a microgravity environment is ensured.

Furthermore, if the gravitational fluctuation stress loading method of the first aspect of the present disclosure is realized by, for example, an aircraft, hypergravity is produced while the aircraft is restoring its airframe orientation. In this case, a time long enough to restore the airframe orientation can be ensured while the hypergravity is being controlled to about 1.3 G to about 2.0 G, which would otherwise be hypergravity of 2.0 G to 2.5 G.

As a result, the first stress-loading step and the second stress-loading step can be repeated alternately without having to apply high load with hypergravity larger than required to the subject or the laboratory animal in the second stress-loading step.

A further aspect according to the present disclosure is provided a method for promoting serotonin-producing gene expression using the above-described gravitational fluctuation stress loading method. This method includes a stress-response producing step of producing a stress response in the subject or the laboratory animal by performing the first stress-loading step; and a gene expression step of selectively expressing a serotonin-producing gene in a cell of the subject or the laboratory animal.

According to the method for promoting serotonin-producing gene expression of the present disclosure, the above-described new stress response produced by physical irritation in the form of microgravity can be made to act specifically on the serotonin-producing gene of chromosomal DNA to selectively express the serotonin-producing gene. Thus, this method can be used for various experiments using a serotonin-producing gene.

A second aspect according to the present disclosure is a serotonin-producing method using the above-described method for promoting serotonin-producing gene expression. This method includes a production step of producing serotonin in the cell by performing the gene expression step.

According to the serotonin-producing method of the second aspect of the present disclosure, because it is possible to selectively produce serotonin that acts on the central nervous system by selectively expressing the serotonin-producing gene, this method can be used as a tool for elucidating the mechanism of how serotonin affects the nervous system, much of which remains to be clarified, by conducting in-vivo analysis.

A further aspect according to the present disclosure is a method for stimulating a central nervous system using the above-described serotonin-producing method. This method includes a nerve stimulation step of stimulating a central nervous system of the subject or the laboratory animal by using the above-described serotonin produced by performing the above-described production step.

According to the method for stimulating a central nervous system of the present disclosure, it is possible to stimulate the central nervous system through serotonin-specific stimulation using selectively produced serotonin.

Furthermore, according to the method for stimulating the central nervous system of the present disclosure, a specimen of a laboratory animal whose central nervous system has been stimulated by the above-described serotonin-specific stimulation can be used as a specimen for elucidating the mechanism causing panic disorder and anxiety disorder of the central nervous system, which are diseases allegedly related to serotonin.

Furthermore, with specimens and measurements collected from the subject or laboratory animal as a tool, it is possible not only to search for drug target molecules that act on the nervous system but also to develop new drugs. In particular, this method can be used for screening drugs effective for serotonin-related diseases of the central nervous system.

A further aspect according to the present disclosure is an efficacy-measuring method using the above-described gravitational fluctuation stress loading method. This method includes a stress-response producing step of producing a stress response in the subject or the laboratory animal by performing the first stress-loading step; a drug administration step of administering a drug to the subject or the laboratory animal; and a measurement step of measuring the efficacy of the drug.

According to the efficacy-measuring method of the present disclosure, if drug target molecules that are likely to provide advantageous effects by acting on the nervous system are discovered or developed, it is possible to observe and measure the action and effects of the drug target molecules upon the nervous system by administering a drug containing the drug target molecules to the subject or the laboratory animal, serving as an examination subject, in which the above-described new stress response has been produced.

Because this allows the efficacy of the drug target molecules upon the nervous system to be checked, it can be used as a new tool for searching for and proposing drug target molecules effective for diseases of the nervous system, such as panic disorder and anxiety disorder.

### Advantageous Effects of Invention

The present invention provides the advantage of causing a new acute stress reaction in a subject or a laboratory animal. It also provides the advantage of selectively expressing a genetic system that works so as to produce/retain serotonin, serving as a neurotransmitter, with the above-described new acute stress reaction.

### Brief Description of Drawings

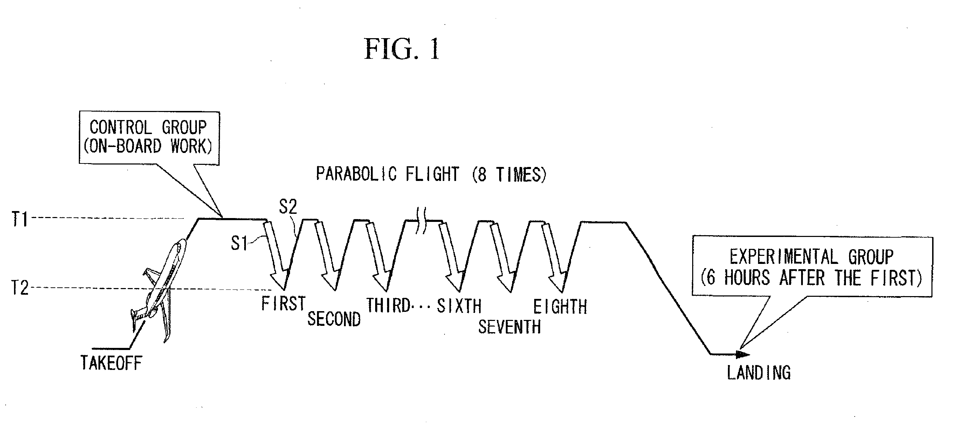

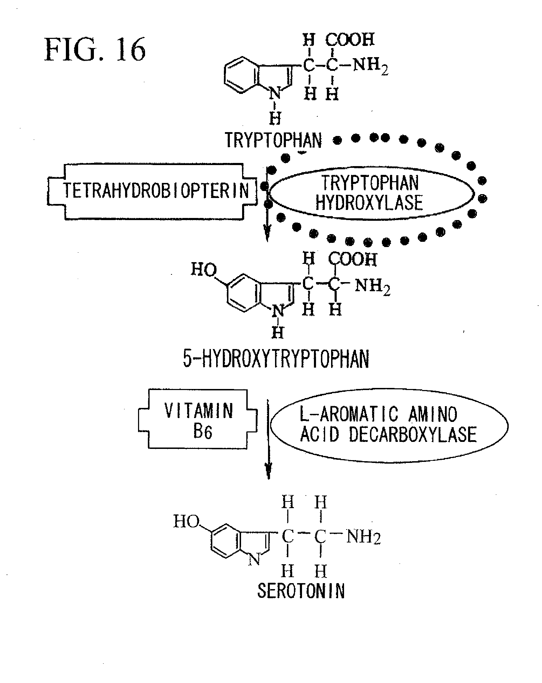

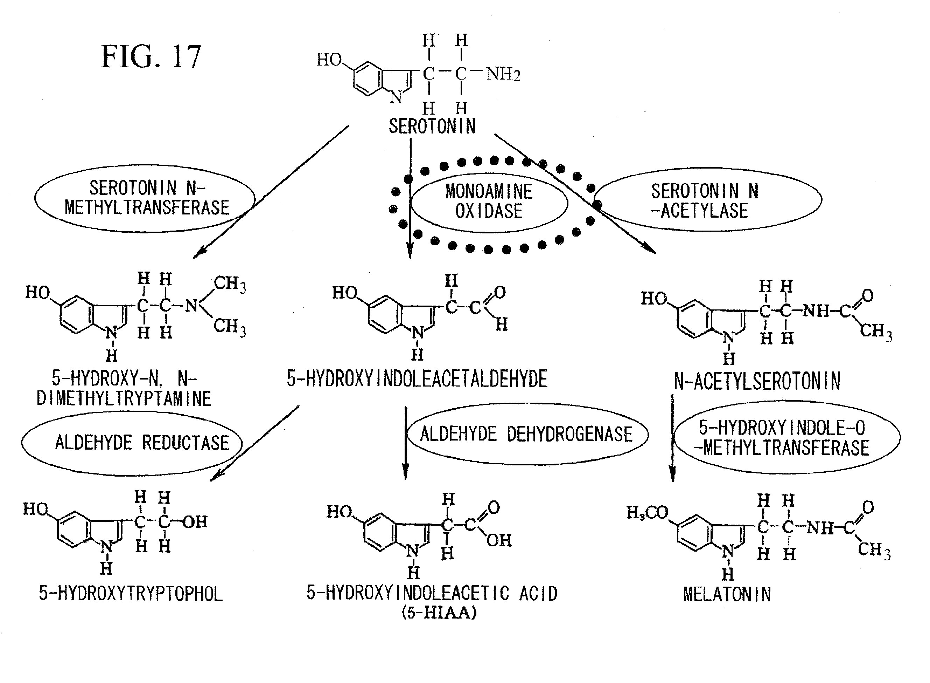

{Fig. 1}
   Fig. 1 is an illustration depicting parabolic flight according to a first embodiment of the present invention.
{Fig. 2}
   Fig. 2 is a diagram depicting environmental acceleration data on the parabolic flight in Fig. 1.
{Fig. 3}
   Fig. 3 is an illustration depicting a stress loading method based on acrophobia.
{Fig. 4}
   Fig. 4 is a graph showing differences in serum corticosterone in blood based on a gravitational fluctuation stress loading method according to the present disclosure.
{Fig. 5}
   Fig. 5 is a table describing results of analyzed gene expression based on stress loading.
{Fig. 6A}
   Fig. 6A is a graph showing changes in gene expression based on the gravitational fluctuation stress loading method according to the present invention.
{Fig. 6B}
   Fig. 6B is a graph showing changes in gene expression based on the gravitational fluctuation stress loading method according to the present invention.
{Fig. 7A}
   Fig. 7A is a graph showing changes in gene expression based on the gravitational fluctuation stress loading method according to the present invention.
{Fig. 7B}
   Fig. 7B is a graph showing changes in gene expression based on the gravitational fluctuation stress loading method according to the present invention.
{Fig. 8A}
   Fig. 8A is a graph showing changes in gene expression based on the gravitational fluctuation stress loading method according to the present invention.
{Fig. 8B}
   Fig. 8B is a graph showing changes in gene expression based on the gravitational fluctuation stress loading method according to the present invention.
{Fig. 9A}
   Fig. 9A is a graph showing changes in gene expression based on the gravitational fluctuation stress loading method according to the present invention.
{Fig. 9B}
   Fig. 9B is a graph showing changes in gene expression based on the gravitational fluctuation stress loading method according to the present invention.
{Fig. 10A}
   Fig. 10A is a graph showing changes in gene expression based on the gravitational fluctuation stress loading method according to the present invention.
{Fig. 10B}
   Fig. 10B is a graph showing changes in gene expression based on the gravitational fluctuation stress loading method according to the present invention.
{Fig. 11A}
   Fig. 11A is a graph showing changes in gene expression based on a stress loading method for acrophobia stress.
{Fig. 11B}
   Fig. 11B is a graph showing changes in gene expression based on a stress loading method for acrophobia stress.
{Fig. 12A}
   Fig. 12A is a graph showing changes in gene expression based on a stress loading method for acrophobia stress.
{Fig. 12B}
   Fig. 12B is a graph showing changes in gene expression based on a stress loading method for acrophobia stress.
{Fig. 13A}
   Fig. 13A is a graph showing changes in gene expression based on a stress loading method for acrophobia stress.
{Fig. 13B}
   Fig. 13B is a graph showing changes in gene expression based on a stress loading method for acrophobia stress.
{Fig. 14A}
   Fig. 14A is a graph showing changes in gene expression based on a stress loading method for acrophobia stress.
{Fig. 14B}
   Fig. 14B is a graph showing changes in gene expression based on a stress loading method for acrophobia stress.
{Fig. 15A}
   Fig. 15A is a graph showing changes in gene expression based on a stress loading method for acrophobia stress.
Fig. 15B}

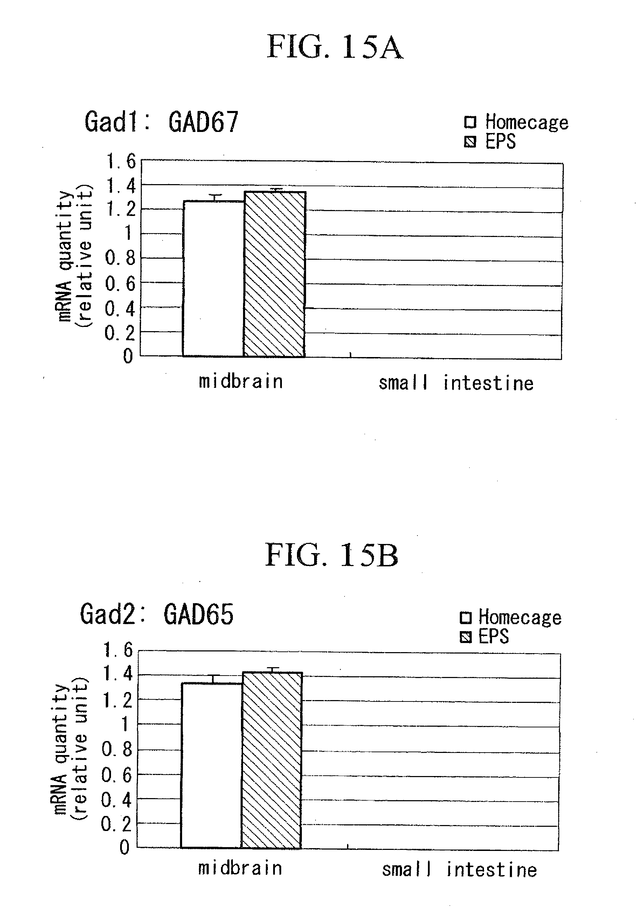

   Fig. 15B is a graph showing changes in gene expression based on a stress loading method for acrophobia stress.
{Fig. 16}
   Fig. 16 is an illustration for a serotonin-producing system.
{Fig. 17}
   Fig. 17 is an illustration for a serotonin metabolic system.

### Description of Embodiments

Embodiments according to the present invention will now be described with reference to the drawings.

### First embodiment

A first aspect according to the present invention is described below.

This embodiment relates to a gravitational fluctuation stress loading method in which a laboratory animal, for example, is subjected to physical irritation in the form of gravitational fluctuation resulting from the parabolic flight of an aircraft.

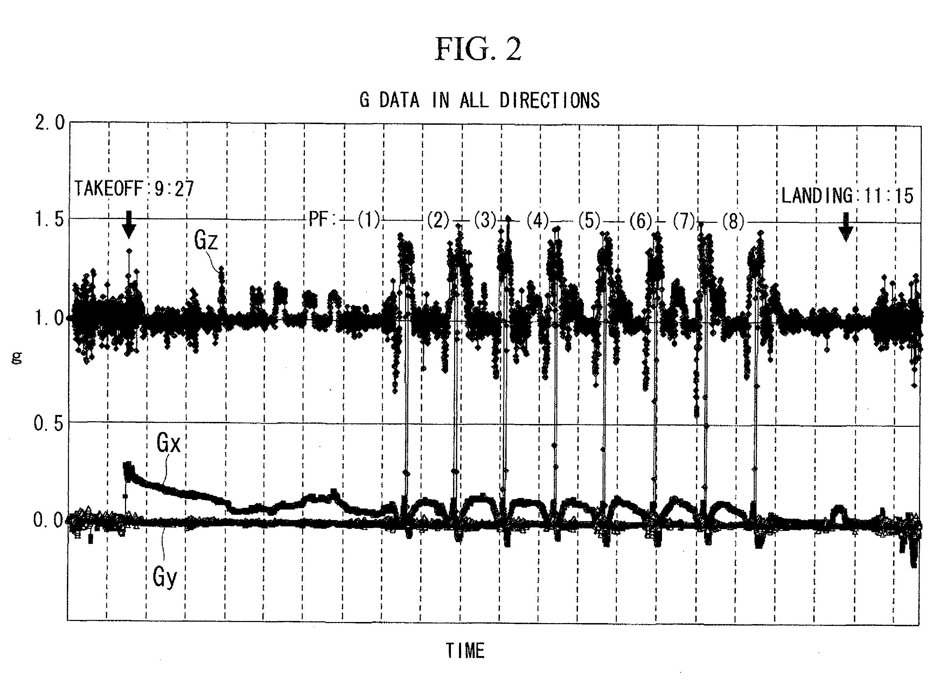

This aspect will be described with reference to Figs. 1 and 2.

Fig. 1 is an illustration of a gravitational fluctuation stress loading method using the parabolic flight of an aircraft.

In this aspect, mice are used as laboratory animals. The aircraft with these mice on board takes off, climbs to a predetermined height (parabolic flight start height T1), and maintains that height. The mice to be examined are placed in a box. This box is retained in a predetermined secure position in the aircraft, such as on a seat, with, for example, a belt.

After the aircraft reaches the predetermined height and the airframe is stabilized, the aircraft starts parabolic flight at parabolic flight start height T1. The aircraft descends continuously through parabolic flight until it reaches a predetermined height (first stress-loading step S1). One parabolic flight continues for about 15 seconds to ensure a sufficient microgravity environment. The microgravity in this microgravity environment is about 10⁻² G.

After the aircraft descends to the predetermined height, the parabolic flight is stopped (parabolic flight stop height T2), and then the aircraft airframe gradually reascends towards the parabolic flight start height T1 while restoring the airframe orientation by changing the aircraft nose direction from downward to upward (second stress-loading step S2). While the aircraft is climbing, a hypergravity environment is formed in the aircraft. The hypergravity in this hypergravity environment is about 1.4 G.

As shown in Fig. 1, the aircraft that has taken off climbs to the parabolic flight start height T1 and then starts the first stress-loading step S1 a second time. In this manner, the aircraft repeats the first stress-loading step S1 and the second stress-loading step S2, eight times each, and then gradually descends from the parabolic flight start height T1 to the ground. It takes about one hour to repeat the above-described first stress-loading step S1 and second stress-loading step S2 eight times each.

Fig. 2 is a diagram depicting environmental acceleration induced while the first stress-loading step S1 and second stress-loading step S2 are repeated, eight times each, by the method shown in Fig. 1. The vertical axis represents aircraft acceleration, and the horizontal axis represents time. The horizontal axis is graduated in six-minute increments.

Furthermore, acceleration Gx, acceleration Gy, and acceleration Gz represent the acceleration in the front/back direction, horizontal direction, and up/down direction, respectively, relative to the aircraft travelling direction.

As shown in Fig. 2, the acceleration in the horizontal direction, denoted by acceleration Gy, is substantially zero from takeoff to landing of the aircraft, indicating that the aircraft travels straight without swaying to the left or right relative to the travelling direction.

For the acceleration in the up/down direction denoted by acceleration Gz, the acceleration temporarily exhibits a slight decrease just before the start of parabolic flight, and during the parabolic flight in the first stress-loading step S1, the downward acceleration increases, causing a sharp rise in the graph. Subsequently, when the parabolic flight stop height T2 is reached, the acceleration temporarily becomes substantially zero. Thereafter, while the aircraft is climbing to the parabolic flight start height T1 restoring the airframe orientation in the second stress-loading step S2, the upward acceleration increases, causing a rise in the graph. As the aircraft is climbing, the upward acceleration gradually decreases, and when the aircraft reaches the parabolic flight start height T1, the acceleration becomes substantially equivalent to that before the start of the parabolic flight.

The acceleration in the front/back direction, denoted by acceleration Gx, is substantially zero because the aircraft moves mainly in the up/down direction relative to the travelling direction, during parabolic flight in the first stress-loading step S1 and during restoration of the airframe orientation and while the aircraft is climbing in the second stress-loading step S2.

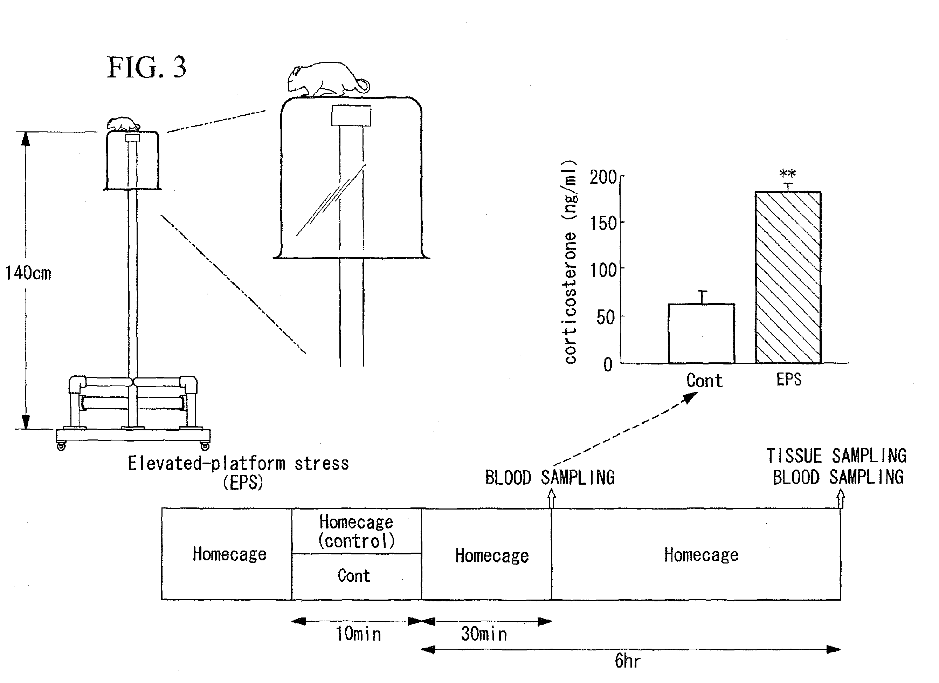

Fig. 3 is an illustration of a stress loading method based on acrophobia stress (EPS: Elevated-platform stress) for use as a comparative controlled experiment.

A transparent cylindrical member is put on a footed rod-shaped member with a height of about 140 centimeters to serve as a circular foothold, on which a mouse taken out of a cage is placed so as to be subjected to acrophobia stress for ten minutes.

After having been subjected to 10-minute acrophobia stress as described above, the mouse is replaced in the cage. Thirty minutes after the mouse is replaced in the cage, blood is collected to measure the blood corticosterone concentration. Corticosterone is a known hormone whose production is promoted under stress by the adrenal cortex.

As shown in Fig. 3, the blood corticosterone concentration of the mouse that has been subjected to the 10-minute acrophobia stress significantly increases compared with that of a mouse in the control group without acrophobia stress. This confirms that the mouse was under acrophobia stress.

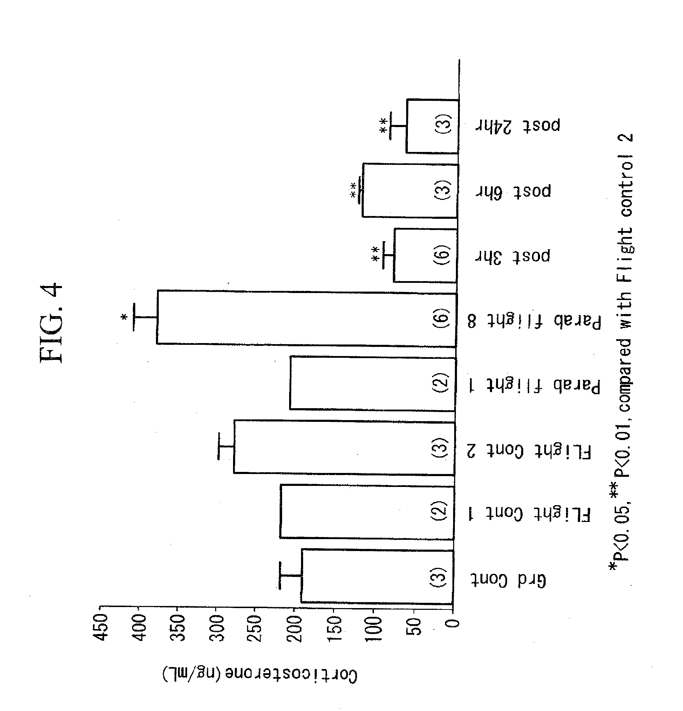

Fig. 4 is a graph of the blood corticosterone concentration of mice (C57BLK/6J, male, 8 weeks old) after being subjected to stress loading by the gravitational fluctuation stress loading method based on the parabolic flight shown in Fig. 1.

More specifically, Fig. 4 shows measurements of the blood corticosterone concentration of mice after being subjected to one parabolic flight (Parab flight 1) and eight parabolic flights (Parab flight 8) by the gravitational fluctuation stress loading method shown in Fig. 1, as well as measurements three hours (post 3hr), six hours (post 6hr), and 24 hours (post 24hr) after the end of those parabolic flights. As controls, the blood corticosterone concentration of mice on the ground (Grd Cont), as well as the blood corticosterone concentration of mice in the aircraft before the start of parabolic flight (Flight Cont), were measured. Figures in parentheses indicate the number of measured individuals.

Fig. 4 demonstrates that the blood corticosterone concentration measured after eight parabolic flights significantly increased, compared with Flight Cont 2, which was the blood corticosterone concentration of mice measured in the aircraft before the start of parabolic flight. This indicates that the mice were under parabolic flight stress.

Furthermore, it was found that the mice were relieved from parabolic flight stress more than three hours after the end of the parabolic flight because the blood corticosterone concentration significantly decreased.

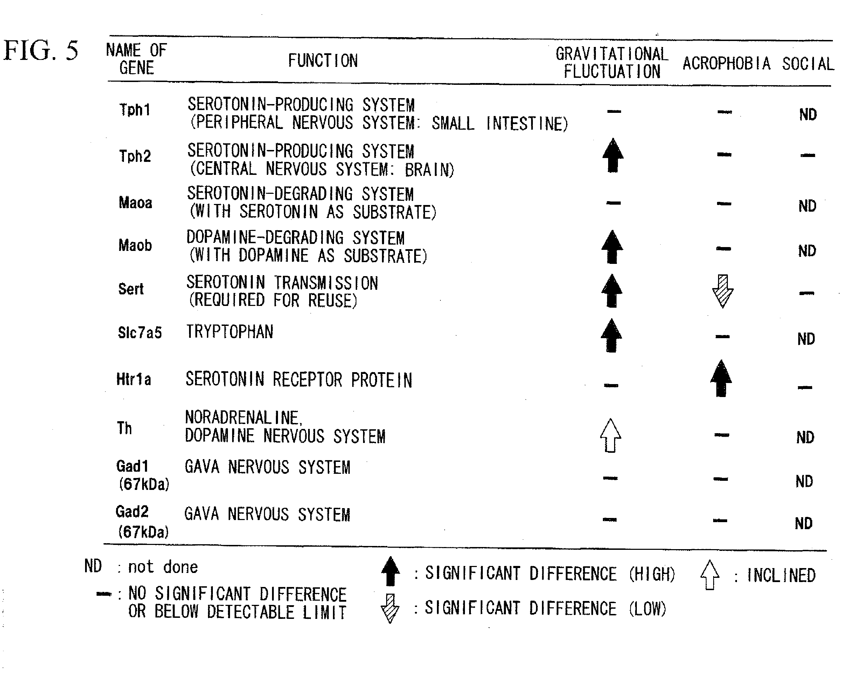

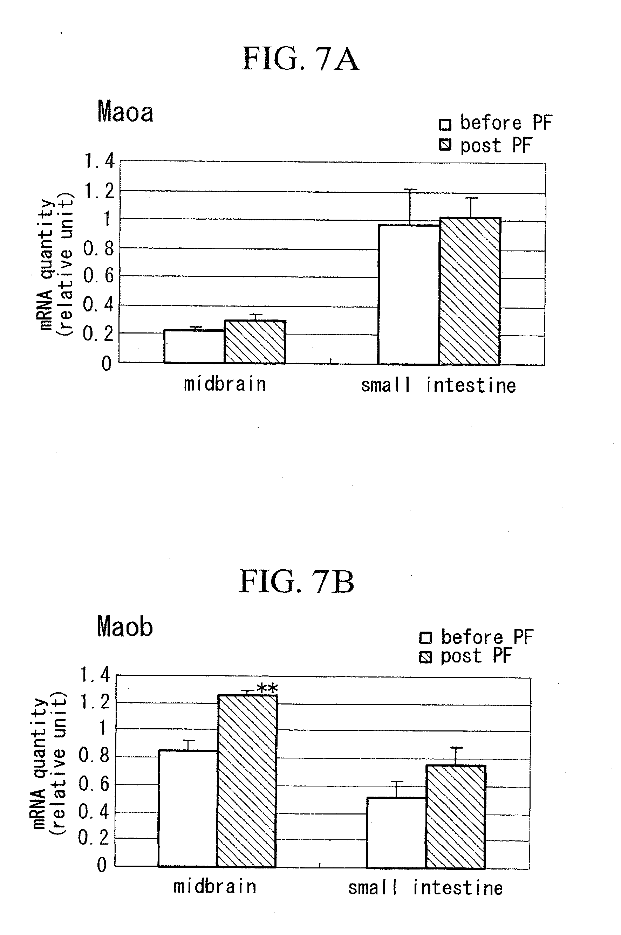

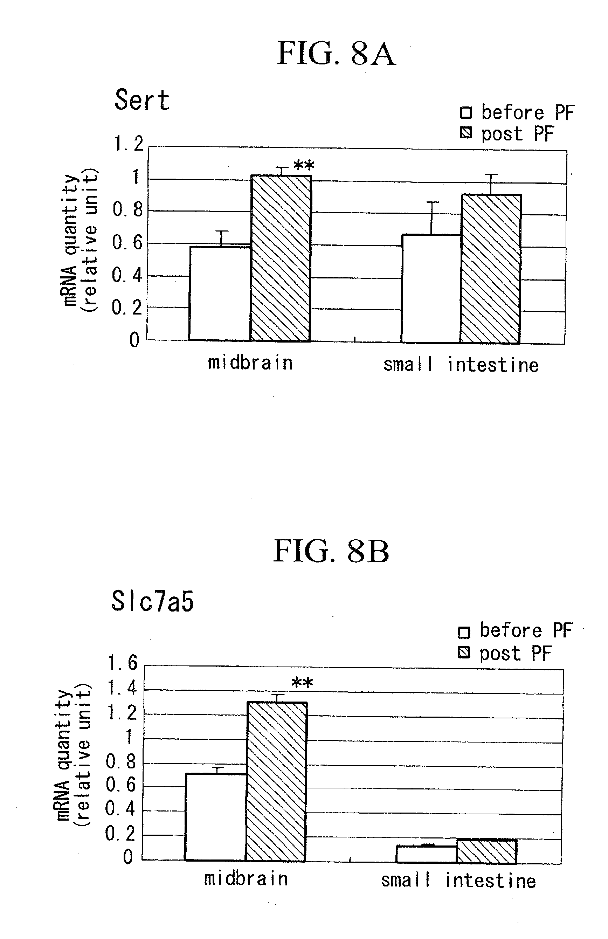

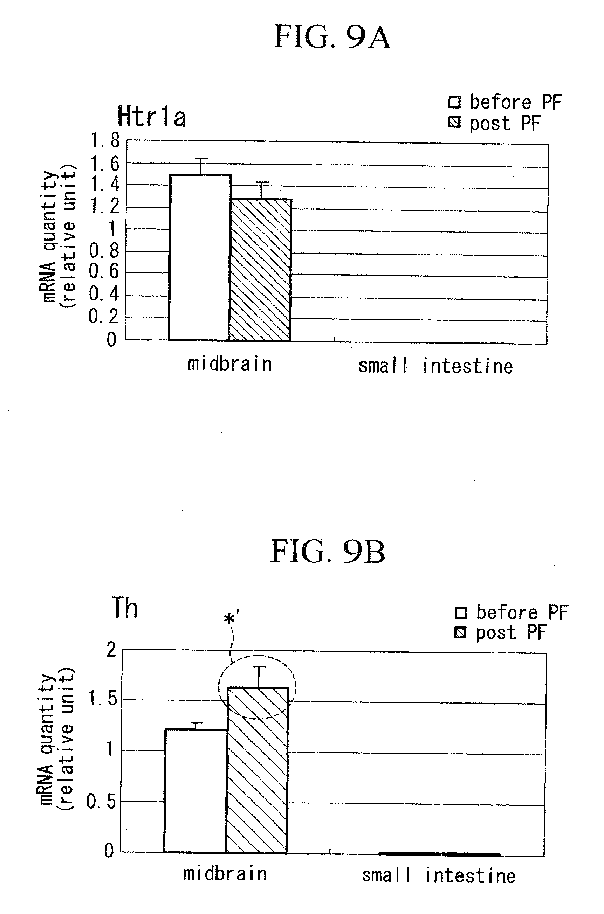

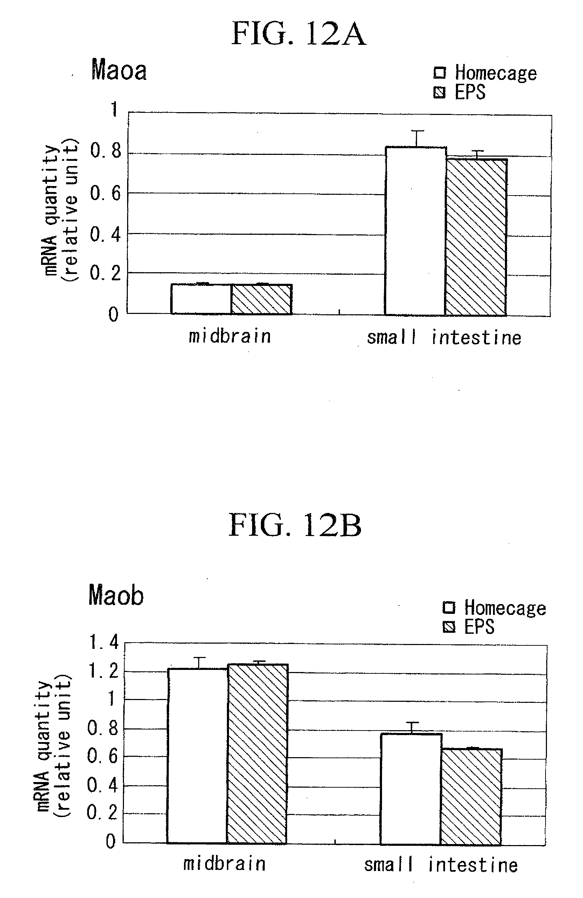

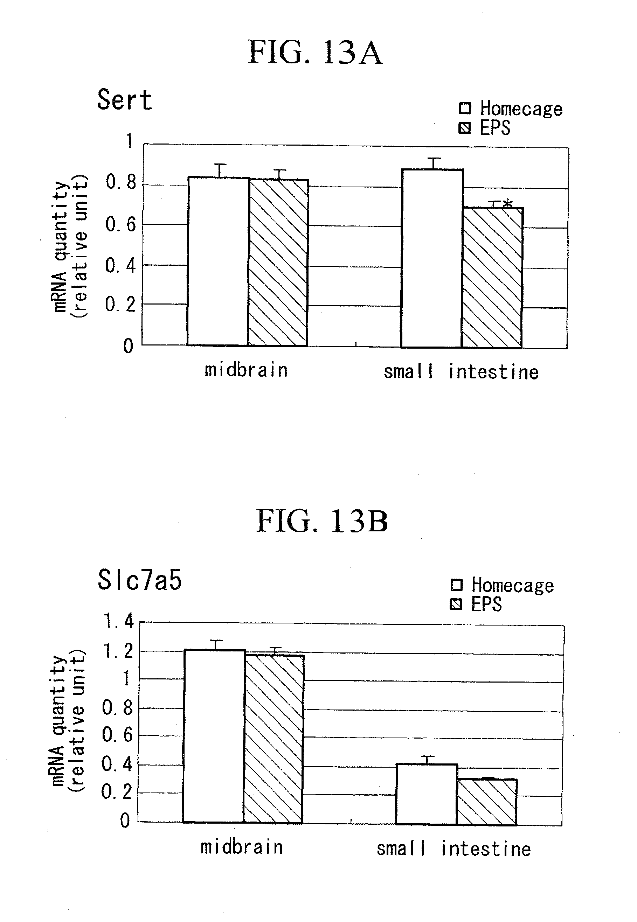

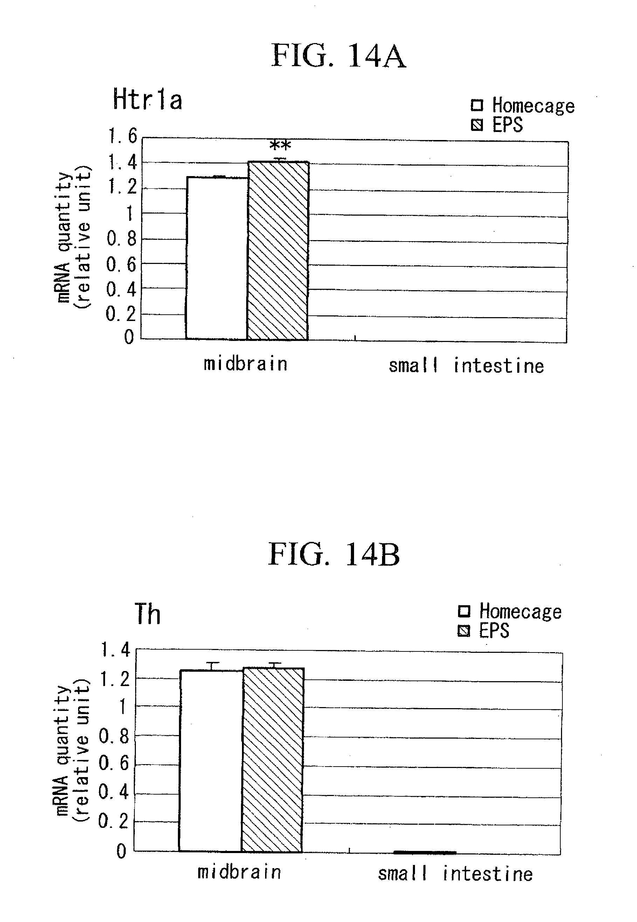

Changes in gene expression due to stress loading will be described with reference to Figs. 5 to 17.

Fig. 5 is a table showing a comparison of intracellular gene expression as influenced by the gravitational fluctuation stress shown in Fig. 1, the acrophobia load stress shown in Fig. 3, and social stress (not shown).

Black arrows pointing upward in Fig. 5 indicate that gene expression after stress loading significantly increased compared with gene expression before stress loading.

White arrows pointing upward in the drawing indicate that gene expression after stress loading increased, though not significantly, compared with gene expression before stress loading.

Arrows pointing downward in the drawing indicate that gene expression after stress loading significantly decreased compared with gene expression before stress loading.

Minus symbols (-) indicate that gene expression was below the detectable limit or exhibited no significant differences. ND indicates that the gene in question was not analyzed.

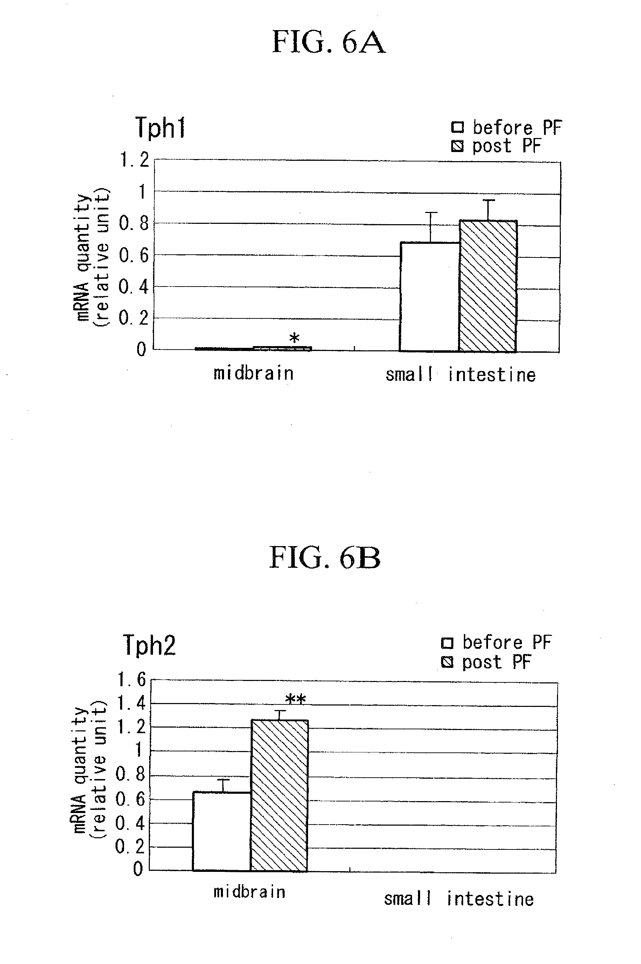

Figs. 6A to 10B are graphs showing changes in gene expression at the mid brain (mid brain), typifying the central nervous system, and at the small intestine (small intestine), typifying the peripheral nervous system, between before and after the gravitational fluctuation stress loading shown in Fig. 1.

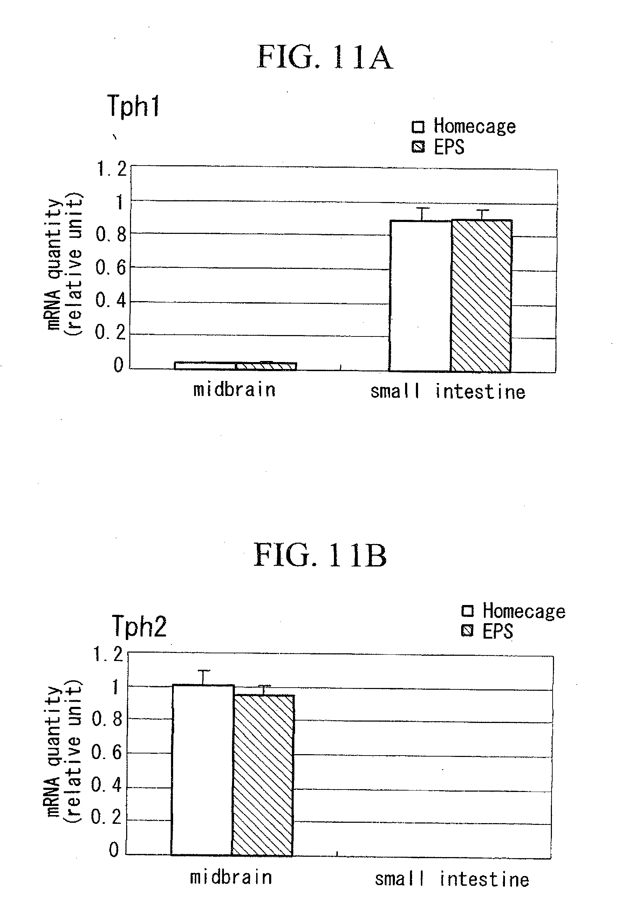

Figs. 11A to 15B are graphs showing changes in gene expression at the mid brain and the small intestine between before and after the acrophobia load stress loading shown in Fig. 3.

The analysis of gene expression before and after the gravitational fluctuation stress loading shown in Fig. 1, as well as the analysis of gene expression before and after the acrophobia load stress loading shown in Fig. 3, was carried out by quantitatively calculating the RNA expression level from a real-time PCR reaction by the intercalator method using SYBR Premix Ex Taq II (manufactured by Takara Bio Inc), with cDNA synthesized for the target gene and the reference gene as a template.

The results of gene expression in the column marked "SOCIAL" in Fig. 5 indicate gene expression of the target gene in the serotonin nucleus (DRN: dorsal raphe nucleus) of a laboratory rat analyzed by the subtractive hybridization method after the laboratory rat was subjected to chronic social stress by bringing the rat into contact with a large, fierce brown rat every day for five weeks (cited from Abumaria, et al. "Identification of Genes Regulated by Chronic Social Stress in the Rat Dorsal Raphe Nucleus," Cellular and Molecular Neurobiology, March 2006, 26 (2) p. 145-162).

The results of gene expression analysis in the column marked "GRAVITATIONAL FLUCTUATION" in Fig. 5 indicate the results of genetic analysis of a mouse that was subjected to stress loading by parabolic flight based on the gravitational fluctuation stress loading method shown in Fig. 1.

More specifically, they are results of analysis derived from measurements of gene expression in cells of the mid brain and the small intestine of the mouse, conducted six hours after completing the first of the eight parabolic flights, subsequent to the landing of the aircraft that performed parabolic flight based on the gravitational fluctuation stress loading method shown in Fig. 1 (post PF in Figs. 6A to 10B). Each parabolic flight lasted for about 15 seconds. Measurement was carried out after six hours in order to ensure a sufficiently long time for gene expression.

In addition, gene expression in cells of a mouse (C57BLK/6J, male, eight weeks old) was analyzed at the stable height the first time the aircraft that had taken off reached the parabolic flight start height T1, and these analysis results were employed as analysis results before parabolic flight (before PF in Figs. 6A to 10B).

The results of gene expression analysis in the column marked "ACROPHOBIA" in Fig. 5 indicate the genetic analysis results of a mouse subjected to stress loading by the acrophobia stress loading method shown in Fig. 3.

More specifically, the mouse was subjected to 10-minute acrophobia stress by the method shown in Fig. 3, the mouse was then replaced in the cage, and gene expression in cells of the mid brain including the raphe nucleus, as well as in cells of the small intestine including chromaffin cells, of the mouse was analyzed six hours after the mouse was replaced in the cage (EPS in Figs. 11A to 15B).

Furthermore, gene expression of a mouse left in the cage was analyzed, and these results were employed as analysis results before acrophobia stress loading (Homecage in Figs. 11A to 15B).

Tph1 (tryptophan hydroxylase 1) is known to be a gene that is related to tryptophan hydroxylase, which acts on the peripheral nervous system, such as the small intestine. Tph2 (tryptophan hydroxylase 2) is known to be a gene that is related to tryptophan hydroxylase, which acts on the central nervous system, such as the brain. Both Tph1 and Tph2 are known to be serotonin-producing genes.

Fig. 16 is a diagram illustrating a serotonin-producing system. As shown in Fig. 16, because serotonin is produced by the effect of tryptophan hydroxylase, serotonin production will be promoted in the peripheral nervous system, such as the small intestine, when Tph1 expression is increased.

Likewise, serotonin production will be promoted in the central nervous system, such as the brain, when Tph2 expression is increased.

Figs. 5, 6A, and 11A demonstrate that the gene expression level of Tph1 in the mid brain and in the small intestine changes only slightly whether by gravitational fluctuation stress loading or by acrophobia stress loading.

In contrast, Figs. 5, 6B, and 11B demonstrate that the gene expression level of Tph2 in the mid brain and the small intestine exhibits no change in response to acrophobia stress loading, whereas gene expression of Tph2 is significantly increased only in the mid brain in response to gravitational fluctuation stress loading. Gene expression of Tph2 also exhibits no change in response to social stress loading.

From the above-described results, it is concluded that gravitational fluctuation stress loading specifically increases Tph2 expression, which acts on the central nervous system, in the mid brain.

Consequently, according to this embodiment, it is possible to produce a new stress response and selectively promote serotonin production in the brain through gravitational fluctuation stress loading using an aircraft.

Maoa (Monoamine oxidase A) is known to be a gene that is related to a serotonin-degrading enzyme with serotonin as a substrate. Maob (Monoamine oxidase B) is known to be a gene that is related to a dopamine-degrading enzyme with dopamine as a substrate.

Fig. 17 is a diagram illustrating a serotonin metabolic system. As shown in Fig. 17, because serotonin is metabolized by the effect of serotonin-degrading enzyme Maoa with serotonin as a substrate, an increase in the expression of Maoa will result in promoting serotonin metabolism.

In addition, because dopamine is metabolized, but serotonin is not metabolized, by the effect of dopamine-degrading enzyme Maob with dopamine as a substrate d, an increase in the expression of Maob will result in promoting only dopamine metabolism.

Figs. 5, 7A, and 12A demonstrate that gene expression of Maoa in the mid brain and the small intestine exhibits no change between before and after the gravitational fluctuation stress loading, nor between before and after the acrophobia stress loading.

In contrast, Figs. 5, 7B, and 12B demonstrate that gene expression of Maob in the mid brain and the small intestine exhibits no change in response to the acrophobia stress loading, whereas gene expression of Maob is significantly increased only in the mid brain in response to the gravitational fluctuation stress loading.

From the above-described results, it is concluded that gravitational fluctuation stress loading specifically increases the expression of dopamine-degrading enzyme Maob in the brain, thereby selectively promoting dopamine metabolism. In short, gene expression is selectively increased so as to cause serotonin not to be metabolized but to be retained. Consequently, according to this embodiment, it is possible to produce a new stress response and selectively promote serotonin retention in the brain through gravitational fluctuation stress loading using an aircraft.

Sert (serotonin transporter) is related to a serotonin transmitter and is known to be a gene that is related to serotonin recycling for effective use of produced serotonin.

Slc7a5 (Solute Carrier Family 7 Member 5) is known to be a large neutral amino acid transporter. Furthermore, tryptophan is known to be a material for producing serotonin. Tryptophan is taken up into cells by the effect of the above-described transporter and is subjected to enzyme reaction in the cells to produce serotonin.

As shown in Figs. 5, 8A, and 13A, as far as gene expression of Sert in the mid brain is concerned, the gene expression level exhibits no change in response to the acrophobia stress loading, whereas gene expression is significantly increased in response to the gravitational fluctuation stress loading.

As far as gene expression of Sert in the small intestine is concerned, the expression level exhibits no change in response to the gravitational fluctuation stress loading, whereas gene expression is significantly decreased in response to the acrophobia stress loading.

Furthermore, social stress loading causes no change in gene expression of Sert.

As shown in Figs. 5, 8B, and 13B, as far as gene expression of Slc7a5 in the mid brain is concerned, the gene expression level exhibits no change in response to the acrophobia stress loading, whereas gene expression is significantly increased in response to the gravitational fluctuation stress loading.

As far as gene expression of Slc7a5 in the small intestine is concerned, the gene expression level exhibits no change between before and after the gravitational fluctuation stress loading, nor between before and after the acrophobia stress loading.

The above-described results demonstrate that gravitational fluctuation stress loading specifically increases the gene expression level of Sert in the brain, thereby selectively promoting reuse of serotonin. Furthermore, because gene expression of Sert decreases in the small intestine, gene expression is increased so as to increase the reuse rate of serotonin in the brain, thus specifically stimulating the central nerve.

Consequently, according to this embodiment, it is possible to produce a new stress response in a mouse, serving as a laboratory animal, and selectively promote the reuse rate of serotonin in the brain through gravitational fluctuation stress loading using an aircraft.

Furthermore, the above-described results demonstrate that gene expression of Slc7a5 is specifically increased in the brain, and thereby extracellular tryptophan is taken up into cells, thus selectively promoting production of serotonin. In addition, because gene expression of Slc7a5 does not change in the small intestine, gene expression is increased so as to produce serotonin in the brain and specifically stimulate the central nervous system.

Therefore, according to this embodiment, it is possible to produce a new stress response by gravitational fluctuation stress loading with an aircraft to selectively promote the production, retention, and reuse rate of serotonin in the brain, thus stimulating the central nervous system in a specific pathway, namely, in a serotonin-selective pathway.

As a result, this embodiment can be used as a tool for elucidating the mechanism allegedly causing serotonin-related diseases of the central nervous system, such as panic disorder or anxiety disorder. In particular, this embodiment can be used for screening drugs effective for serotonin-related diseases of the central nervous system.

Htr1a (serotonin 1A receptor) is known to be a gene that is related to a serotonin receptor that receives produced serotonin.

Th (tyrosine hydroxylase) is known to be a gene that is related to the first-stage synthetase of thyrosin serving as a material of adrenaline, noradrenaline, and dopamine.

Gad1 (glutamic acid decarboxylase 1), glutamic acid decarboxylase with a molecular weight of 67 kDa, is known to be a gene that is related to GAVA, which is a neurotransmitter that works so as to suppress brain activation.

Gad2 (glutamic acid decarboxylase 2), glutamic acid decarboxylase with a molecular weight of 65 kDa, like Gad1, is known to be a gene that is related to GAVA.

As shown in Figs. 5, 9A, and 14A, as far as gene expression of Htr1a in the mid brain is concerned, the gene expression level exhibits no change in response to the gravitational fluctuation stress loading, whereas gene expression is significantly increased in response to the acrophobia stress loading. Social stress loading causes no change in gene expression of Htr1a.

As far as gene expression of Th in the mid brain is concerned, the expression level exhibits no change in response to the acrophobia stress loading, whereas gene expression increases, though not significantly, in response to the gravitational fluctuation stress loading.

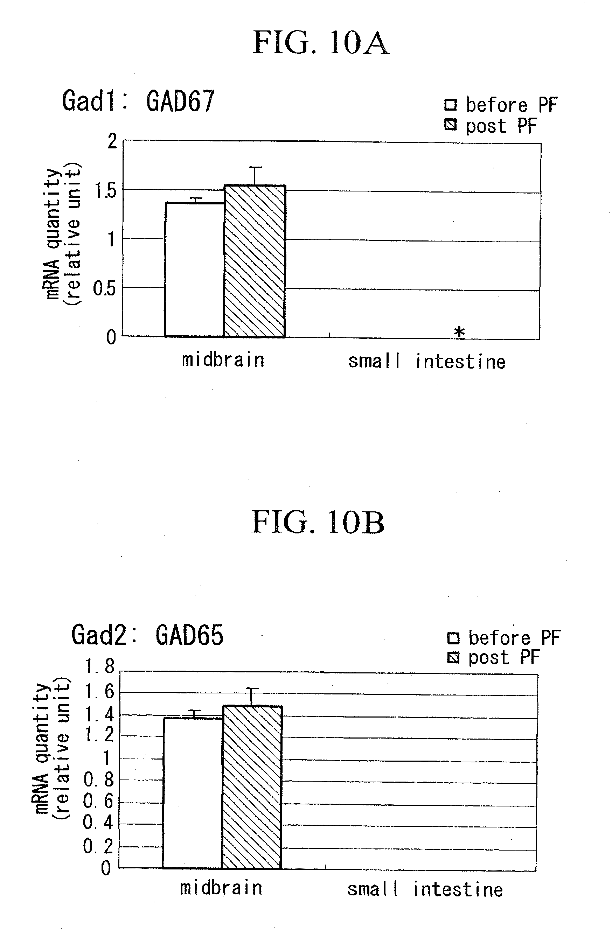

Furthermore, as shown in Figs. 10A, 10B, 15A, and 15B, gene expression of Gad1 and Gad2 in the mid brain and the small intestine exhibit no change between before and after the gravitational fluctuation stress loading, nor between before and after the acrophobia stress loading.

The above-described results demonstrate that because gravitational fluctuation stress loading is likely to increase gene expression of Th in the brain, it is also likely to activate the entire nervous system by promoting activation of the adrenaline nervous system, the noradrenaline nervous system, the dopamine nervous system, and so forth. However, because the gene expression degree of Th does not exhibit a significant difference, only a minor influence on gene expression will result, though gene expression is somewhat likely to increase.

The results described above demonstrate that, through gravitational fluctuation stress loading with an aircraft according to this embodiment, it is possible to produce a new stress response, which was impossible to produce by conventional stress loading methods.

Although the first stress-loading step S1 and the second stress-loading step S2 are repeated eight times each in this embodiment, the number of repetitions is not limited to this. The first stress-loading step S1 may be carried out, for example, only once. This is because forming a microgravity environment at least once can afford the advantage of the present application. Furthermore, the first stress-loading step S1 and the second stress-loading step S2 may be repeated more than eight times each.

Furthermore, although gravitational fluctuation stress loading, including the first stress-loading step S1 and the second stress-loading step S2, takes about one hour in this embodiment, the stress loading time is not limited to this. The stress loading time may be more or less than one hour, depending on the number of times that the first stress-loading step S1 and the second stress-loading step S2 are repeated.

Although the microgravity in the first stress-loading step S1 is about 10⁻² G in this embodiment, the microgravity is not limited to this. The microgravity may be less than 1 G ranging, for example, from 10⁻⁶ G to 10⁻² G.

In addition, although the hypergravity in the second stress-loading step S2 is about 1.4 G in this embodiment, the hypergravity is not limited to this. The hypergravity may range, for example, from 1 G to 2.5 G.

Furthermore, although the parabolic flight start height T1 and the parabolic flight stop height T2 are predetermined heights in this embodiment, T1 and T2 are not limited to this. The start height of the first parabolic flight may be different from those of the second and subsequent parabolic flights, and furthermore, the stop height of the first parabolic flight may be different from those of the second and subsequent parabolic flights, as long as a microgravity environment can be maintained in the aircraft for a predetermined period of time.

Furthermore, although the durations of the first to eighth parabolic flights are constant in this embodiment because the parabolic flight start height T1 and the parabolic flight stop height T2 are predetermined heights, the durations of the parabolic flights are not limited to this. For example, the duration of the first parabolic flight may be 15 seconds, and the durations of the second and subsequent parabolic flights may be 20 seconds. Furthermore, the parabolic flight duration may be less than 15 seconds, for example, 10 seconds, and is not limited particularly.

### Second embodiment

A second embodiment according to the present invention will now be described.

This embodiment relates to an efficacy-measuring method for measuring the efficacy of a drug which has been administered to a laboratory animal, such as a mouse, or a subject serving as an examination subject after the examination subject has been subjected to gravitational fluctuation stress loading according to the first embodiment.

Drugs to be administered include, but are not limited to, drugs including drug target molecules that have been found to be likely to effectively act on depression, panic disorder, anxiety disorder, and other diseases of the nervous system.

Items to be measured over time include, but are not limited to, the blood concentration of drug target molecules after drug administration. Specimens that can be collected from the above-described examination subject, such as blood, cells, chromosomal DNA and protein, or other measurable parameters are also acceptable.

Methods for measuring the drug efficacy include, but are not limited to, the use of measuring devices, such as a gas chromatograph mass spectrometer (GC-MS), a liquid chromatograph (HPLC), and a flow cytometer. Any kinds of known measuring devices can be combined. Furthermore, the drug efficacy may be measured to obtain radioactivity using drug target molecules at least one of whose substituents is labeled with a radioactive label, such as ¹²⁵I, ³H, or ¹⁴C.

According to this embodiment, it is possible to observe and measure the effect of drug target molecules of a drug upon the nervous system of a laboratory animal or a subject after the drug has been administered to the laboratory animal or the subject in which a new stress response is produced by the gravitational fluctuation stress loading method according to the first embodiment.

Because this allows the efficacy of the drug containing drug target molecules to be studied and developed, this embodiment can be used as a new tool for finding and proposing drug target molecules effective for diseases of the nervous system, such as panic disorder and anxiety disorder.

The present invention is not limited to the above-described embodiments but can be modified as necessary without departing from the spirit and the scope of the present invention.

### Reference Signs List

- S1: first stress-loading step
- S2: second stress-loading step
- T1: parabolic flight start height
- T2: parabolic flight stop height

## Claims

1. A method of promoting gene expression of Tph2, Maob, Sert and Slc7a5 in a mid-brain of a non-human laboratory animal comprising performing a gravitational stress loading method which comprises:
at least one first stress-loading step of placing a stress load on the non-human laboratory animal by means of microgravity, and
at least one second stress-loading step of placing a stress load on the non-human laboratory animal by means of hypergravity.

2. The method according to Claim 1, wherein the first stress-loading step and the second stress-loading step are repeated alternately.

3. The method according to Claims 1 or 2, wherein the first stress-loading step is performed not more than 20 times in one hour.

4. The method according to claim 1 wherein a microgravity environment and hypergravity environment are formed in an aircraft through a parabolic flight.

5. An efficacy-measuring method using the method according to one of Claims 1 to 4, comprising:
a stress-response producing step of producing a stress response in the non-human laboratory animal by performing the first stress-loading step and the second stress-loading step;
and
a drug screening step

## Patentansprüche

1. Verfahren zum Fördern der Genexpression von Tph2, Maob, Sert und Slc7a5 im Mittelhirn eines nicht-menschlichen Labortiers, umfassend das Durchführen eines Schwerkraftstress-Belastungsverfahrens, das umfasst:
mindestens einen ersten Schritt der Stressbelastung durch Aussetzen eines nicht-menschlichen Labortiers gegenüber einer Stressbelastung mittels Mikroschwerkraft, und
mindestens einen zweiten Schritt der Stressbelastung durch Aussetzen eines nicht-menschlichen Labortiers gegenüber einer Stressbelastung mittels Hyperschwerkraft.

2. Verfahren nach Anspruch 1, wobei der erste Schritt der Stressbelastung und der zweite Schritt der Stressbelastung alternierend wiederholt werden.

3. Verfahren nach Anspruch 1 oder 2, wobei der erste Schritt der Stressbelastung nicht mehr als 20-mal in einer Stunde wiederholt wird.

4. Verfahren nach Anspruch 1, wobei eine Mikroschwerkraftumgebung und eine Hyperschwerkraftumgebung in einem Flugzeug durch parabolischen Flug entstehen.

5. Wirksamkeitsmessverfahren unter Verwendung des Verfahrens nach einem der Ansprüche 1 bis 4, umfassend
einen Schritt der Stress-Response-Erzeugung zum Erzeugen einer Stressantwort in dem nicht-menschlichen Labortier durch Ausführen des ersten Schritts der Stressbelastung und des zweiten Schritts der Stressbelastung; und
einen Schritt des Screenens auf Arzneimittel.

## Revendications

1. Procédé pour promouvoir l'expression génique de Th2, Maob, Sert et Slc7a5 dans le mésencéphale d'un animal de laboratoire non humain comprenant à mise en oeuvre d'une méthode de soumission à un stress gravitationnel qui comprend :
au moins une première étape de soumission à une charge de stress consistant à créer une charge de stress chez l'animal de laboratoire non humain en le plaçant en microgravité, et
au moins une seconde étape de soumission à une charge de stress consistant à créer une charge de stress chez l'animal de laboratoire non humain en le plaçant en hypergravity.

2. Procédé selon la revendication 1, dans lequel la première étape de soumission à une charge de stress et la seconde étape de soumission à une charge de stress sont répétées alternativement.

3. Procédé selon les revendications 1 ou 2, dans lequel la première étape de soumission à une charge de stress n'est pas exécutée plus de 20 fois par heure.

4. Procédé selon la revendication 1 dans lequel un environnement de microgravité et un environnement d'hypergravité sont créés dans un avion en vol parabolique.

5. Procédé de mesure d'efficacité utilisant le procédé selon l'une des revendications 1 à 4, comprenant :
une étape de production de réponse à une charge de stress consistant à produire une réponse à une charge de stress chez l'animal de laboratoire non humain par exécution de la première étape de soumission à une charge de stress et de la seconde étape de soumission à une charge de stress, et
une étape de criblage de médicaments.
